# EUROPÄISCHE PATENTSCHRIFT

(11) **EP 0 415 872 B1**
(45) Veröffentlichungstag und Bekanntmachung des Hinweises auf die Patenterteilung: **12.07.1995**
(21) Anmeldenummer: 90710020.0
(22) Anmeldetag: 27.07.1990
(51) Int. Cl.: A61F 2/28, A61F 2/30, B22C 9/04, A61F 2/00

(54) **Verfahren zur Herstellung eines Implantates mit einer seine Oberfläche zumindest teilweise bedeckenden metallischen, offenzelligen Struktur**
Method for producing an implant having a surface covered at least partially by a metallic open-cell structure
Procédé pour la fabrication d'un implant comportant une surface couvert au moins partiellement d'une structure métallique à pores ouvertes

(30) Priorität: 28.08.1989 DE 3928394
(43) Veröffentlichungstag der Anmeldung: 06.03.1991
(73) Patentinhaber: ESKA Implants GmbH & Co., 23556 Lübeck (DE)
(72) Erfinder: Ahlers, Olaf, D-2000 Hamburg 79 (DE)
(74) Vertreter: Weiss, Christian, Dipl.-Ing.

(56) Entgegenhaltungen:
- EP-A- 0 399 163
- DE-A- 2 112 320
- DE-A- 3 224 265
- GB-A- 2 093 701
- US-A- 3 890 107

## Beschreibung

Die Erfindung betrifft ein Verfahren zur Herstellung eines Implantates mit einer seine Oberfläche zumindest teilweise bedeckenden metallischen, offenzelligen Struktur gemäß dem Oberbegriff des Ansprüches 1.

Die damit hergestellten Implantate ermöglichen das Einwachsen von Knochenmaterial in die offenzellige Oberflächenstruktur, wodurch für deren dauerhafte Fixation im Knochen gesorgt wird.

Das gattungsgemäße Verfahren ist aus der Praxis bekannt und geht zurück beispielsweise auf ein Verfahren gemäß der DE-A-32 24 265. Darin wird vorgeschlagen, als Verstärkungsmittel für die Wandungen und/oder Stege des porigen Kunststofformkörpers Wachs zu verwenden, welches entweder in flüssiger Form oder als Bestandteil einer Wasser-Wachs-Emulsion in den Kunststofformkörper eingebracht wird.

Nach dem Trocknen des Wachses auf den Wandungen und/oder Stegen wird dieses durch eine Kunststofflackbeschichtung geschützt.

Als nachteilig hat sich bei diesem Verfahren erwiesen, daß sich das mittels Eintauchen oder durch Einsprühen auf bzw. in den Kunststofformkörper gebrachte Wachs in Verbindung mit dem aufgesprühten Lack gegenüber dem elastischen Kunststofformkörper starr verhält, so daß sich das Wachs unter Druck leicht von den Wandungen und Stegen der Poren des Formkörpers lösen kann. Ein weiterer wesentlicher Nachteil dieses Auftragverfahrens ist darin zu sehen, daß sich die Polymeroberflächen der Wandungen und Stege des Kunststofformkörpers negativ gegenüber dem Wachs oder der Wachs-Wasser-Emulision aufladen. Infolgedessen kommt es zu keinem zirkulär-homogenen Verbund zwischen den Wandungen und Stegen des Kunststofformkörpers und dem Wachs. Insbesondere trifft dies zu auf die Wandungen und Stege, die sich in der räumlichen Tiefe des Formkörpers, also von dessen Oberfläche entfernt befinden. Das Fließverhalten des Wachses oder der Wachs-Wasser-Emulsion in dem Formkörper ist also nicht so günstig, als daß auch die Wandungen und Stege in der räumlichen Tiefe des Positivmodells in befriedigendem Maße verstärkt werden. In der Folge weist dementsprechend der mit dem Verfahren hergestellte metallische Formkörper in seiner räumlichen Tiefe Wandungen und Stege auf, die teilweise zu dünn sind, um auftretenden Belastungen dauerhaft standhalten zu können.

Vor dem aufgezeigten Hintergrund ist es nun die Aufgabe der vorliegenden Erfindung, das eingangs erwähnte Verfahren so weiterzubilden, daß die Wandungen und Poren des auf einem Wachsgrundkörper verhafteten offenporigen Kunststoffträgers durchgängig, das heißt also auch in dessen räumlicher Tiefe zuverlässig verstärkt werden, wobei darilber hinaus die Veraschung des als Verstärkungsmittel verwendeten Materials problemlos möglich sein soll.

Gelöst wird diese Aufgabe durch den Verfahrensschritt gemäß den kennzeichnenden Teilen der Ansprüche 1 und 2.

Demgemäß wird der Kunststoffträger nach erfolgter Reinigung zur Verstärkung seiner Wandungen und/oder vernetzten Stege zumindest einmal durchgängig entweder mit einer Lösung von Polymethylmetacrylat (PMMA), oder alternativ hierzu mit einer Lösung von Polyesterharz als Verstärkungsmittel in einem Lösungsmittel besprüht.

Die Reinigung des Kunststoffträgers kann vorzugsweise mittel Aceton vorgenommen werden.

Wird PMMA als Verstärkungsmittel verwendet, eignet sich als Lösungsmittel ebenfalls Aceton. Wird hingegen Polyesterharz zur Verstärkung der Wandungen und/oder Stege verwendet, kann beispielsweise eine Nitrolösung als Lösungsmittel dienen.

Das Lösungsmittel wird in beiden Fällen die Oberflächenstrukturen des Kunststoffträgers anlösen und sich relativ rasch verflüchtigen. Das jeweils verwendete Verstärkungsmittel bleibt auf der Oberflächenstruktur des Kunststoffträgers als verstärkende Schicht zurück. Durch das vorherige Anlösen der Oberflächenstrukturen durch das Lösungsmittel wird eine regelrechte Sogwirkung erzeugt, wodurch das verwendete Verstärkungsmittel auch in räumlich tiefer gelegene Schichten des Kunststoffträgers, aber auch im Sprühschatten liegende Bereiche vordringen kann. Die auf den Oberflächenstrukturen des Kunststoffträgers zurückbleibende Schicht des Verstärkungsmittels aus PMMA oder Polyesterharz lassen sich problemlos im weiteren Verlauf des Verfahrens veraschen.

Wird eine relativ dicke Verstärkungsschicht der Wandungen und/oder Stege gewünscht, wird zweckmäßigerweise so vorgegangen, daß der Kunststoffträger mehrfach durchgängig mit der Lösung des jeweiligen Verstärkungsmittels in einem Lösungsmittel nach Erstarren der jeweils vorangehend aufgebrachten Schicht des Verstärkungsmittels besprüht wird. Hierbei findet also eine regelrechte Aufschichtung des verwendeten Verstärkungsmittels statt.

Es kann auch so vorgegangen werden, daß nach dem Erstarren der zuletzt aufgebrachten Verstärkungsschicht der Kunststoffträger durchgängig mit einem die Oberflächenstrukturen der letzten Verstärkungsschicht anlösenden haftungsvermittelnden Harzfilm benetzt wird und anschließend mindestens eine Schicht eines selbstvernetzenden Zweikomponenten-Silicons durchgängig in bzw. auf den Kunststoffträger ein- bzw. aufgetragen wird. Hierdurch wird eine bizarre Oberflächenstruktur des Positivmodells erreicht, welche sich entsprechend in dem Endprodukt wiederfindet. Diese bizarre Oberflächenstruktur ist erwünscht für eine gewisse Aggressivität hinsichtlich der Reizung des nach der Implantation des Implantates an der offenzelligen Struktur anliegenden Knochenmaterials, wodurch letztendlich ein beschleunigtes Einwachsen von Knochenmaterial in die Struktur erreicht wird.

Zur zusätzlichen Versteifung der wie vorbeschrieben erzeugten bizarren Oberflächenstruktur des im Kunststoffträger befindlichen Verstärkungsnetzwerkes kann ein Zweikomponenten-Polyurethanharz auf bzw. eingetragen werden. Zur Herstellung eines innigen Verbundes zwischen dem Zweikomponenten-Silicon und dem aufzutragenden Zweikomponenten-Polyurethanharz hat das letztere aufgrund seiner Zusammensetzung eine die Oberfläche des Zweikomponenten-Silicons leicht anlösende Eigenschaft.

Der haftungsvermittelnde Harzfilm zwischen den PMMA- bzw. Polyesterharzschichten und dem Zweikomponenten-Silicon besteht bei Ausführung der vorerwähnten Verfahrensweise vorzugsweise aus einem ungesättigten Siliconharz. Hierdurch ergibt sich ein System, bei dem ein ungesättigtes Siliconharz dem ein- bzw. aufzutragenen Zweikomponenten-Silicon gegenübersteht, dessen Gemisch regelmäßig ebenfalls ungesättigt sein wird. Hierdurch wird wieder eine regelrechte Saugwirkung (Anziehung) zwischen dem Silikonharz und dem Zweikomponenten-Silicon erzielt, wodurch in besonders eindrucksvoller Weise die Stege und Wandungen der Poren des Kunstoffträgers auch in dessen räumlicher Tiefe verstärkt werden.

Das Zweikomponenten-Silicon ist vorteilhafterweise ein raumtemperaturvernetzender, ungesättigter Zweikomponenten-Silicon-Kautschuk. Grundsätzlich kann die Vernetzung natürlich auch durch Abschrecken das mit dem Zweikomponenten-Silicon versehenen Kunststoffträgers in Wasser erzielt bzw. beschleunigt werden.

Oftmals wird die Notwendigkeit bestehen, die Wandungen und/oder die Stege der Poren des Kunststoffträgers durch Auf- bzw. Eintragen mehrerer Schichten des PMMA bzw. Polyesterharzes und gegebenenfalls des Zweikomponenten-Silicons zu verstärken. Hierbei sind die einzelnen Schichten vorteilhafterweise verschiedenfarbig. Dies ermöglicht es, die Schichtdicken beispielsweise unter einer Lupe erkennbar zu machen, um die Gleichmäßigkeit der Schichtdicken kontrollieren zu können. Diese Kontrolle dient der Sicherung der Qualität der mit dem erfindungsgemäßen Verfahren hergestellten offenzelligen metallischen Strukturen. Eine gleichmäßige Verstärkung der Wandungen und/oder vernetzten Stege der Poren des Kunststoffträgers spiegelt sich nach Durchführung des Verfahrens wider in der Gleichmäßigkeit der Wandungen und/oder Stege der metallischen Struktur.

Die Lösung des PMMA oder des Polyesterharzes im Lösungsmittel sollte vorzugsweise eine Temperatur unterhalb von 12°C haben. Der Grund hierfür ist darin zu sehen, daß die Lösungsmittel für PMMA und Polyesterharz, beispielsweise Aceton bzw. Nitrolösung, einen recht niedrigen Verdampfungspunkt haben. Die verwendete Lösung wird ein Mischungsverhältnis im Bereich von 75:25 (Lösungsmittel: PMMA bzw. Polyesterharz) aufweisen, damit sie leicht versprüht werden kann, beispielsweise mittels einer Spritzpistole. Bei höheren Temperaturen, etwa bei Raumtemperatur, erfolgt eine solch rasche Verdampfung des Lösungsmittels, daß sich die Konzentrationsverhältnisse zu schnell ändern würden, um den Verfahrensschritt des Besprühens - insbesondere bei Serienherstellung von Implantaten - durchführen zu können.

Die durchschnittliche Weite der Poren des Kunststoffträgers wird vorzugsweise im Bereich von 1 bis 3 mm liegen. Gegenüber einem nach dem eingangs erwähnten bekannten Verfahren hergestellten Implantat als Knochenersatz weist die mit vorliegendem Verfahren hergestellte metallische Struktur größere Poren und an ihrer Außenseite schärfere Kanten auf. Die größeren Poren ermöglichen aufgrund ihrer Dimensionierung das Einwachsen von Knochenbälkchen auch in ihre räumliche Tiefe hinein. Die Scharfkantigkeit an der Außenseite ergibt sich daraus, daß die Wahrscheinlichkeit eines Durchtrennens der größeren Poren bei dem Zuschnitt des Kunststoffträgers des Positivmodells wesentlich größer ist als beim Zuschnitt eines Kunststoffträgers mit kleineren Poren.

## Patentansprüche

1. Verfahren zur Herstellung eines Implantates mit einer seine Oberfläche zumindest teilweise bedeckenden metallischen offenzelligen Struktur unter Anwendung eines verlorenen Positivmodells aus einem Wachsgrundkörper und einem auf diesem verhafteten offenporigen Kunststoffträger mit durch ein Besprühen mit einem Verstärkungsmittel verstärkten Wandungen und/oder vernetzten Stegen, bei dem
- das Positivmodell im Ganzen in einer keramischen Masse eingebettet wird,
- das Modell erhitzt wird, wobei der Wachsgrundkörper schmilzt, der Kunststoffträger mit seiner Verstärkung verascht und die keramische Masse gebrannt wird
- in die Zurückbleibenden Keruräume Metall gegossen wird, und
- nach dessen Erstatten die keramische Masse entfernt wird,
dadurch gekennzeichnet,
daß als Verstärkungsmittel des Kunstsoffträgers nach erfolgter Reinigung in einem die Oberflächenstrukturen des Kunstsoffträgers anlösenden Lösungsmittel gelöstes Polymethylmetacrylat verwendet wird und zumindest einmal durchgängig besprüht wird.

2. Verfahren zur Herstellung eines Implantates mit einer seine Oberfläche zumindest teilweise bedeckenden metallischen offenzelligen Struktur unter Anwendung eines verlorenen Positivmodells aus einem Wachsgrundkörper und einem auf diesem verhafteten offenporigen Kunststoffträger, mit durch ein Besprühen mit einem Verstärkungsmittel verstärkten Wandungen und/oder vernetzten Stegen, bei dem
- das Positivmodell im Ganzen in einer keramischen Masse eingebettet wird,
- das Modell erhitzt wird, wobei der Wachsgrundkörper schmilzt, der Kunststoffträger mit seiner Verstärkung verascht und die keramische Masse gebrannt wird,
- in die zurückbleibenden Hohlräume Metall gegossen wird, und
- nach dessen Erstarren die keramische Masse entfernt wird,
dadurch gekennzeichnet, daß als Verstärkungsmittel des Kunstsoffträgers nach erfolgter Reinigung in einem die Oberflächenstrukturen des Kunststoffträgers anlösenden Losungsmittel gelöstes Polyesterharz verwendet wird und zumindest einmal durchgängig besprüht wird.

3. Verfahren nach Anspruch 1 oder 2, dadurch gekennzeichnet, daß der Kunststoffträger mehrfach durchgängig mit der Lösung des Verstärkungsmittels in einem Lösungsmittel nach Erstarren der jeweils vorangehend aufgebrachten Schicht des Verstärkungsmittels besprüht wird.

4. Verfahren nach einem der Ansprüche 1 bis 3, dadurch gekennzeichnet, daß nach dem Erstarren der zuletzt aufgebrachten Verstärkungsschicht der Kunststoffträger durchgängig mit einem die Oberflächenstrukturen der genannten Schicht anlösenden haftungsvermittelnden Harzfilm benetzt wird, und daß anschließend mindestens eine Schicht eines selbstvernetzenden Zweikomponenten-Silicons durchgängig in bzw. auf den Kunststoffträger ein- bzw. aufgetragen wird.

5. Verfahren nach Anspruch 4, dadurch gekennzeichnet, daß nach der Vernetzung des Zweikomponenten-Silicons die Oberflächenstruktur zusätzlich durch ein Zweikomponenten-Polyurethanharz versteift wird.

6. Verfahren nach Anspruch 4, dadurch gekennzeichnet, daß der haftungsvermittelnde Harzfilm aus einem ungesättigten Siliconharz besteht.

7. Verfahren nach einem der Ansprüche 4 bis 6, dadurch gekennzeichnet, daß das Zweikomponenten-Silicon ein raumtemperaturvernetzender, ungesättigter Zweikomponenten-Siliconkautschuk ist.

8. Verfahren nach einem der Ansprüche 1 bis 7, dadurch gekennzeichnet, daß die einzelnen aufgebrachten Verstärkungsschichten jeweils unterschiedlich gefärbt sind.

9. Verfahren nach einem der Ansprüche 1 bis 8, dadurch gekennzeichnet, daß die Lösung des Verstärkungsmittels in dem Lösungsmittel eine Temperatur unterhalb von 12°C hat.

## Claims

1. Process for producing an implant having a metallic open-pore structure at least partially covering its surface using a lost positive model of a wax base and an open-pore plastic support adhered to the latter, and having walls and/or crosslinked bars reinforced by spraying with a reinforcing agent, in which
- the entire positive model is embedded in a ceramic composition,
- the model is heated, wherein the wax base melts, the plastic support and its reinforcement is incinerated and the ceramic composition is fired,
- metal is cast into the remaining core spaces, and
- after it is solidified, the ceramic composition is removed,
characterised in that dissolved polymethylmethacrylate is used as reinforcing agent for the plastic support after cleaning is completed in a solvent which partially dissolves the surface structures of the plastic support, and is thoroughly sprayed at least once.

2. Process for producing an implant having a metallic open-pore structure at least partially covering its surface using a lost positive model of a wax base and an open-pore plastic support adhered to the latter, and having walls and/or crosslinked bars reinforced by spraying with a reinforcing agent, in which
- the entire positive model is embedded in a ceramic composition,
- the model is heated, wherein the wax base melts, the plastic support and its reinforcement is incinerated and the ceramic composition is fired,
- metal is cast into the remaining hollow spaces, and
- after it is solidified, the ceramic composition is removed,
characterised in that dissolved polyester resin is used as reinforcing agent for the plastic support after cleaning is completed in a solvent which partially dissolves the surface structures of the plastic support, and is thoroughly sprayed at least once.

3. Process according to claim 1 or 2, characterised in that the plastic support is sprayed thoroughly several times with the solution of the reinforcing agent in a solvent after solidifying the particular layer of reinforcing agent applied beforehand.

4. Process according to one of claims 1 to 3, characterised in that after solidifying the reinforcing layer applied last, the plastic support is wetted thoroughly with an adhesion-promoting resin film which partially dissolves the surface structures of the said layer, and in that at least one layer of a self-crosslinking two-component silicone is then applied thoroughly into or onto the plastic support.

5. Process according to claim 4, characterised in that after crosslinking the two-component silicone, the surface structure is additionally stiffened by means of a two-component polyurethane resin.

6. Process according to claim 4, characterised in that the adhesion-promoting resin film consists of an unsaturated silicone resin.

7. Process according to one of claims 4 to 6, characterised in that the two-component silicone is an unsaturated two-component silicone rubber which crosslinks at room temperature.

8. Process according to one of claims 1 to 7, characterised in that the individual reinforcing layers applied each have a different colour.

9. Process according to one of claims 1 to 8, characterised in that the solution of the reinforcing agent in the solvent has a temperature below 12°C.

## Revendications

1. Procédé pour la fabrication d'un implant avec une structure à alvéoles ouvertes métallique recouvrant au moins partiellement sa surface, avec utilisation d'un modèle positif perdu à partir d'un corps de base en cire et d'un support en matière plastique à pores ouverts, attaché au corps de base en cire, avec des parois et/ou des nervures réticulées renforcées par pulvérisation d'un agent de renforcement, dans lequel
- le modèle positif est entièrement noyé dans une masse céramique,
- le modèle est chauffé, ce faisant le corps de base en cire fond, le support en matière plastique est incinéré avec son renforcement et la masse céramique est cuite,
- du métal est coulé dans les espaces de noyau restants, et
- la masse céramique est enlevée après la solidification du métal,
caractérisé en ce que l'on utilise, comme agent de renforcement du support en matière plastique, après nettoyage, du polyméthylméthacrylate dissout dans un solvant dissolvant les structures superficielles du support en matière plastique et qu'on l'asperge au moins une fois dans son entier.

2. Procédé pour la fabrication d'un implant avec une structure à alvéoles ouvertes métallique recouvrant au moins partiellement sa surface, avec utilisation d'un modèle positif perdu à partir d'un corps de base en cire et d'un support en matière plastique à pores ouverts, attaché au corps de base en cire, avec des parois et/ou des nervures réticulées renforcées par pulvérisation d'un agent de renforcement, dans lequel
- le modèle positif est entièrement noyé dans une masse céramique,
- le modèle est chauffé, ce faisant le corps de base en cire fond, le support en matière plastique est incinéré avec son renforcement et la masse céramique est cuite,
- du métal est coulé dans les cavités restantes,
- la masse céramique est enlevée après la solidification du métal,
caractérisé en ce que 'lon utilise, comme agent de renforcement du support en matière plastique et après nettoyage, de la résine polyester dissoute dans un solvant dissolvant les structures superficielles du support en matière plastique et qu'on l'asperge au moins une fois dans son entier.

3. Procédé selon la revendication 1 ou 2, caractérisée en ce que le support en matière plastique est aspergé plusieurs fois et dans son entier avec la solution de l'agent de renforcement dans un solvant, après solidification de la couche d'agent de renforcement qui a été appliquée précédemment.

4. Procédé selon l'une des revendications 1 à 3, caractérisé en ce que, après la solidification de la couche de renforcement qui a été appliquée en dernier, le support en matière plastique est humecté dans son entier par une pellicule de résine, réalisant l'adhérence et dissolvant les structures superficielles de la couche citée, et en ce que, pour finir, au moins une couche d'un silicone à deux composantes réalisant une autoréticulation, est appliquée entièrement dans ou sur le support en matière plastique.

5. Procédé selon la revendication 4, caractérisé en ce que, après la réticulation du silicone à deux composants, la structure superficielle est rigidifiée de façon supplémentaire par une résine polyuréthane à deux composants.

6. Procédé selon la revendication 4, caractérisé en ce que la pellicule de résine réalisant l'adhérence est composée d'une résine silicone insaturée.

7. Procédé selon l'une des revendications 4 à 6, caractérisé en ce que le silicone à deux composants est un caoutchouc de silicone à deux composants insaturé réalisant la réticulation à la température ambiante.

8. Procédé selon l'une des revendications 1 à 7, caractérisé en ce que les diverses couches de renforcement appliquées sont chacune d'une couleur différente.

9. Procédé selon l'une des revendications 1 à 8, caractérisé en ce que la solution de l'agent de renforcement dans le solvant présente une température inférieure à 12 °C.
